(19) Europäisches Patentamt
European Patent Office
Office européen des brevets

(11) Numéro de publication : **0 059 659**
**B1**

(12) # FASCICULE DE BREVET EUROPÉEN

(45) Date de publication du fascicule du brevet : **04.07.84**

(21) Numéro de dépôt : **82400286.9**

(22) Date de dépôt : **18.02.82**

(51) Int. Cl.[3] : **C 07 C 69/708**, C 07 C 69/716, C 07 D307/10, C 07 C 69/757, C 07 C 67/347, C 07 C 67/31, C 07 C 67/313

(54) **Nouveaux dérivés de l'acide 4-méthyl 3-formyl pentanoïque, leur procédé de préparation et leur application à la préparation de dérivés cycliques.**

(30) Priorité : **26.02.81 FR 8103832**

(43) Date de publication de la demande : **08.09.82 Bulletin 82/36**

(45) Mention de la délivrance du brevet : **04.07.84 Bulletin 84/27**

(84) Etats contractants désignés : **AT BE CH DE FR GB IT LI LU NL SE**

(56) Documents cités : **Néant**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140-Bondy (FR)**
Inventeur : **Tessier, Jean**
**30, rue Jean Moulin**
**F-94300-Vincennes (FR)**
Inventeur : **Demoute, Jean-Pierre**
**249 bis, rue de Rosny**
**F-93100-Montreuil sous Bois (FR)**

(74) Mandataire : **Tonnellier, Marie-José et al**
**ROUSSEL-UCLAF 111, route de Noisy Boîte Postale no.9**
**F-93230 Romainville (FR)**

EP 0 059 659 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés de l'acide 4-méthyl 3-formyl pentanoïque, leur procédé de préparation et leur application à la préparation de dérivés cycliques.

L'invention a pour objet les composés de formule (I) :

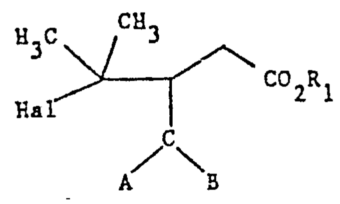

(I)

dans laquelle Hal représente un atome d'halogène, $R_1$ représente un radical alkyle renfermant de 1 à 12 atomes de carbone et le groupement :

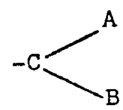

représente soit un groupement :

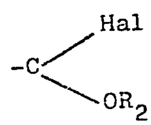

dans lequel Hal est défini comme précédemment et $R_2$ représente un radical alkyle renfermant de 1 à 12 atomes de carbone, soit un groupement :

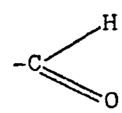

Hal représente de préférence un atome de chlore ou de brome. $R_1$ représente de préférence un radical méthyle, éthyle, propyle, isopropyle, butyle, isobutyle ou pentyle.

$R_2$ représente de préférence un radical méthyle, éthyle, propyle, isopropyle, butyle, isobutyle ou pentyle.

L'invention a donc notamment pour objet les composés de formule (I) répondant à la formule ($I_A$) :

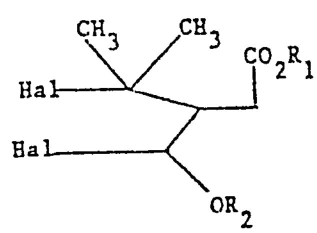

($I_A$)

dans laquelle Hal, $R_1$ et $R_2$ sont définis comme précédemment, ainsi que ceux répondant à la formule ($I_B$) :

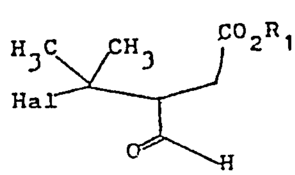

($I_B$)

dans laquelle Hal et $R_1$ sont définis comme précédemment.

L'invention a notamment pour objet les composés de formule (I) pour lesquels Hal représente un atome de chlore ainsi que ceux pour lesquels $R_1$ représente un radical méthyle.

L'invention a également pour objet un procédé de préparation des composés de formule (I), caractérisé en ce que l'on soumet un composé de formule (II)

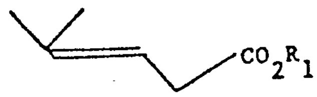

(II)

dans laquelle $R_1$ conserve sa signification précédente à l'action d'un composé de formule (III)

$$(Hal)_2CHOR_2 \qquad (III)$$

dans laquelle Hal et $R_2$ sont définis comme précédemment, en présence d'un agent acide, pour obtenir un composé de formule ($I_A$) :

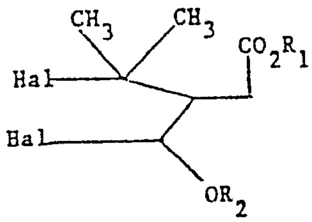

($I_A$)

que l'on soumet, le cas échéant, à l'action d'un agent d'hydrolyse, pour un composé de formule ($I_B$) correspondant :

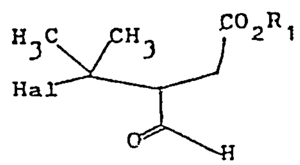

($I_B$)

L'agent acide utilisé peut être un acide minéral comme l'acide chlorhydrique ou sulfurique, mais l'invention a plus précisément pour objet un procédé de préparation tel que défini précédemment, dans lequel l'acide utilisé est un acide de Lewis.

Comme acide de Lewis, on peut citer $AlCl_3$, $SnCl_4$, $TiCl_4$, $FeCl_3$ ou $ZnCl_2$.

L'invention a plus particulièrement pour objet un procédé de préparation, tel que défini précédemment, caractérisé en ce que l'on fait réagir sur un composé de formule (II) :

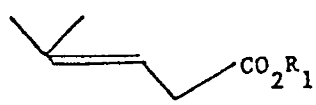

(II)

d'un composé de formule (III) :

$$(Hal)_2CHOR_2 \qquad (III)$$

dans laquelle $R_2$ a la même signification que $R_1$.

Dans un mode de réalisation préféré du procédé de l'invention :

— la réaction entre le composé de formules II et le composé de formule (III) a lieu à basse température, par exemple −80 et 0 °C et de préférence entre −10 °C et −50 °C ;

— la réaction entre le composé de formule II et le composé de formule (III) a lieu au sein du chlorure de méthylène ou de tout autre solvant inerte ;

— l'agent d'hydrolyse est l'eau et l'on opère à basse température, par exemple, entre −50 °C et 10 °C et de préférence au voisinage de 0 °C.

Les produits de formule (I) sont d'un très grand intérêt industriel ; ils sont préparés par un procédé simple et très rapide à partir des composés de formule (II) :

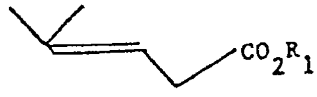

(II)

Il est possible, en fait, comme le montre la partie expérimentale, de préparer en un seul stade, les produits de formule ($I_B$) à partir des produits de formule (II), car il n'est pas nécessaire d'isoler les produits de formule ($I_A$).

La partie expérimentale exposée ci-après met également clairement en évidence la facilité de séparation et de purification des produits de formule ($I_B$) ainsi que des produits mis en œuvre lors de l'application des produits de formule ($I_A$) ou ($I_B$). Il s'agit, en effet, de produits isolables par cristallisation ou distillation.

Les produits de formule (I) peuvent être transformés rapidement pour des réactions très simples, mettant en jeu des réactifs peu coûteux, en produits d'un très grand intérêt industriel, qui permettent la préparation de très nombreux produits pesticides dérivés de l'acide cyclopropane carboxylique de la série cis ou trans, par exemple ceux décrits dans les brevets français 2.185.612.

On ne connaissait pas jusqu'à présent de procédé permettant de préparer des précurseurs d'esters d'acide cyclopropane carboxylique cis ou trans. L'industriel qui souhaitait fabriquer des esters des séries cis et des esters des séries trans devrait « monter » deux unités opérationnelles distinctes. Le procédé de la présente invention permet d'utiliser les mêmes installations pendant une grande partie de la synthèse des esters d'acide cis ou trans. Cela est inattendu puisqu'on ne connaissait pas de procédé permettant d'accéder à la fois aux séries cis et aux séries trans, cela est d'un grand intérêt industriel, car cela permet de diminuer les coûts de production des produits ainsi préparés. La mise en œuvre des produits de formule (I) apporte une solution à un problème industriel jamais résolu à ce jour.

L'invention a donc également pour objet l'application des produits de formule (I) à la préparation de produits utiles pour la synthèse industrielle de dérivés d'acide cyclopropane carboxylique des séries cis ou trans.

L'invention a ainsi pour objet :

a) l'application des composés de formule (I) répondant à la formule ($I_A$), caractérisée en ce que l'on soumet un composé de formule ($I_A$), à l'action d'un agent d'hydrolyse acide, pour obtenir un composé de formule (IV) :

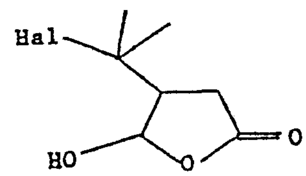

(IV)

dans laquelle Hal est défini comme précédemment ;

b) l'application des composés de formule (I) répondant à la formule ($I_A$), caractérisée en ce que l'on soumet un composé de formule ($I_A$) à l'action d'un alcool de formule (V) :

$$R_2—OH \quad (V)$$

dans laquelle $R_2$ est défini comme précédemment, pour obtenir un composé de formule (VI) :

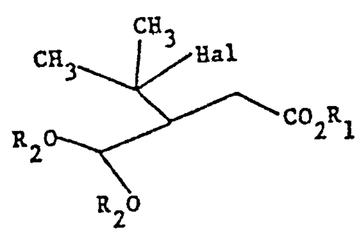

(VI)

que l'on soumet successivement à l'action d'une base, d'un agent de saponification et enfin d'un réactif d'hydrolyse, pour obtenir le composé de formule (VII) :

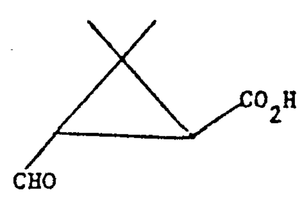

(VII)

de configuration trans ;

c) l'application des composés de formule (I) répondant à la formule ($I_B$), caractérisée en ce que l'on soumet un composé de formule ($I_B$) à l'action d'un alcool $R_2$—OH dans lequel $R_2$ est défini comme

précédemment, pour obtenir un composé de formule (VI) défini précédemment, puis poursuit la synthèse, pour obtenir un composé de formule (VII).

Dans un mode de réalisation préférée de l'application, selon l'invention,

— les réactifs d'hydrolyse conduisant aux composés (IV) et (VII), sont l'acide chlorhydrique ou sulfurique ou tout autre acide fort en solution aqueuse,

— la réaction avec l'alcool $R_2$—OH a lieu entre 5 °C et la température ambiante, l'alcool utilisé est choisi en fonction des valeurs préférées de $R_2$ ;

— la base utilisée est la soude aqueuse concentrée ou tout autre base forte en milieu non aqueux et la réaction est effectuée en présence d'un catalyseur de transfert de phase ;

— l'agent de saponification est un hydroxyde alcalin ou alcalino-terreux en solution hydroalcoolique ;

— l'action de l'alcool sur le composé de formule ($I_B$) a lieu en présence d'un catalyseur acide, par exemple, l'acide paratoluène sulfonique.

Comme il a été indiqué plus haut, les produits obtenus lors de la mise en œuvre des produits de formule (I), selon l'application décrite ci-dessus sont des produits industriels bien connus qui permettent la préparation d'esters d'acide cyclopropane carboxylique des séries cis ou trans utilisés dans la lutte contre les parasites.

Le composé de formule (VII) ou acide 2,2-diméthyl 3-formyl cyclopropane carboxylique, est un produit connu décrit dans le brevet français 2.185.612.

Les composés de formule (IV) sont également des produits connus qui sont décrits et revendiqués dans la demande de brevet français 2.458.533 et qui permettent de préparer notamment l'hémiacylal interne de l'acide 2,2-diméthyl 3-formyl cyclopropane-1-carboxylique de formule :

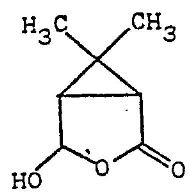

lequel est un produit industriel connu qui permet l'accès aux dérivés de l'acide cyclopropane carboxylique de la série cis, et qui est décrit et revendiqué sous ses formes optiquement actives dans le brevet français 1.580.474.

Les composés de formule (VI) sont des produits chimiques nouveaux.

L'invention a donc pour objet ces produits de formule (VI) à titre de produits industriels nouveaux et plus précisément à titre de produits intermédiaires nécessaires à la préparation des acides 2,2-diméthyl 3-formyl cyclopropane carboxyliques (VII).

Les exemples suivants illustrent l'invention sans toutefois la limiter.

## Exemple 1

4-chloro 4-méthyl 3-formyl pentanoate de méthyle

Stade A : 3-(méthoxy chlorométhyl)4-chloro 4-méthyl pentanoate de méthyle

On refroidit à − 35 °C un mélange renfermant 1,4 g de trichlorure d'aluminium et 10 $cm^3$ de chlorure de méthylène et ajoute 1 $cm^3$ d'$\alpha$-dichlorométhyl méthyl éther dissous dans 5 $cm^3$ de chlorure de méthylène.

On ajoute entre − 40 °C et − 50 °C à la suspension ainsi obtenue, 1 g de 4-méthyl 3-penténoate de méthyle dans 6 $cm^3$ de chlorure de méthylène et agite pendant 1 heure en laissant remonter la température à − 10 °C/0 °C. On obtient ainsi un mélange renfermant le produit recherché en solution chlorométhylénique.

Stade B : 4-chloro 4-méthyl 3-formyl pentanoate de méthyle.

On verse le mélange obtenu au Stade A dans une solution aqueuse glacée de bicarbonate de sodium. On extrait avec du chlorure de méthylène, évapore le solvant et obtient 1,5 g d'un produit que l'on chromatographie sur silice en éluant avec de l'hexane à 20 % d'acétate d'éthyle. On obtient ainsi 600 mg du produit recherché qui cristallise à − 20 °C.

RMN ($CDCl_3$ p.p.m.)

— 1,65 et 1,7 : H des méthyles géminés
— 10 : H du groupement formyle
— 3,2 à 3,4 : H en $\alpha$ du formyle
— 2,33 à 2,95 : H en $\alpha$ de l'ester
— 3,72 : H du méthyle de l'ester

Exemple 2

Application : 4-(2-chloro-prop-2yl)5-hydroxy tetrahydrofuran-2-one

On verse le mélange préparé au stade A de l'exemple 1 dans un mélange d'eau et de glace, agité pendant 1 heure entre 0 et 10 °C et extrait au chlorure de méthylène. On lave à l'aide d'une solution diluée d'acide chlorhydrique, sèche sur sulfate de soude et concentre à sec sous pression réduite sans dépasser 20 ~ 25 °C. On obtient 1,66 g d'une huile que l'on dissout immédiatement dans 20 $cm^3$ d'acétone et ajoute à 20 °C 20 $cm^3$ d'une solution d'acide chlorhydrique N. On agite pendant 20 heures à 20/25 °C. On dilue à l'eau, extrait au chlorure de méthylène pour isoler 1,4 g de cristaux gommeux qui sont purifiés par empatage dans l'éther de pétrole (Eb 40 ~ 70 °C). On obtient ainsi 1,03 g du produit recherché fondant à 83 °C, identique au produit décrit au stade A de l'exemple 5 de la demande de brevet français 2.458.533.

Exemple 3

Application : préparation de l'acide 2,2-diméthyl 3-formyl cyclopropane carboxylique-trans

Stade A : 4-chloro 4-méthyl 3-diméthoxy méthyl pentanoate de méthyle

On maintient sous agitation pendant 1 heure à 20 °C un mélange renfermant 5 g de l'aldéhyde préparé à l'exemple 1,50 $cm^3$ de méthanol et 50 mg d'acide paratoluène sulfonique.
On verse sur une solution aqueuse de carbonate acide de sodium, extrait au chloroforme et rectifie. On obtient ainsi le produit recherché Eb. (0,05 mmHg) = 62 °C.
RMN-$CDCl_3$ p.p.m.
— 1,61 et 1,62 : H des méthyles géminés
— 4,47-4,51 : H de l'acétal,
— 3,34-3,4 : H des radicaux diméthoxy de l'acétal,
— 3,7 : H du méthoxy de l'ester.

Stade B : 1 (RS,trans)3-diméthoxy méthyl 2,2-diméthyl cyclopropane carboxylate de méthyle.

On maintient sous agitation pendant 24 heures à 20 °C ~ 25 °C, un mélange renfermant 716 mg du produit préparé au Stade A, 7 $cm^3$ de chlorure de méthylène, 14 $cm^3$ d'une solution de soude à 50 % et 70 mg de chlorure de triéthylbenzylammonium. On verse le mélange réactionnel dans une solution aqueuse saturée de dihydrogéno-phosphate de sodium, extrait au benzène, sèche sur sulfate de sodium, filtre puis évapore à sec sous pression réduite. On purifie par chromatographie sur silice en éluant par le mélange benzène-acétate d'éthyle 95/5 et obtient 494 mg du produit recherché.

Stade C : Acide 2,2-diméthyl 3-formyl cyclopropane carboxylique-trans

A 1 g du composé préparé au Stade B dissous dans 20 $cm^3$ de méthanol, on ajoute 10 $cm^3$ de soude aqueuse 2 N et on chauffe le mélange deux heures au reflux. Après avoir été refroidi, le milieu réactionnel est acidifié avec suffisamment d'acide chlorhydrique aqueux 6 N pour obtenir une solution à pH 1. Cette solution est abandonnée une heure à 20 °C avant d'être diluée à l'eau. L'extraction au chlorure de méthylène suivie d'une évaporation du solvant fournit l'acide aldéhyde recherché.

Exemple 4

Application : préparation de l'acide 2,2-diméthyl 3-formyl cyclopropane carboxylique-trans.

Le procédé est le même que celui décrit à l'exemple 3 (stade B et C) mais comporte une variante au Stade A.

Stade A : 4-chloro 4-méthyl 3-diméthoxy méthyl pentanoate de méthyle.

On verse le mélange obtenu à l'exemple 1, stade A sur du méthanol à basse température. On agite 3 heures à 5-10 °C et verse sur une solution aqueuse de bicarbonate de soude. On sépare l'insoluble par filtration, on décante le chlorure de méthylène et extrait la phase aqueuse au chlorure de méthylène. On lave les extraits organiques au bicarbonate de soude et évapore à sec sous vide. On obtient 11 g de produit que l'on purifie par chromatographie sur silice en éluant par de l'éther de pétrole (Eb 40 ~ 70 °C) à 10 % d'acétate d'éthyle. On obtient ainsi 8,66 g du produit recherché Eb. (0,05 mm Hg) = 62 °C identique, à celui obtenu au stade A de l'exemple 3.

**Revendications** (pour les Etats contractants : BE, CH, DE, FR, GB, IT, LI, LU, NL, SE)

1. Les composés de formule (I) :

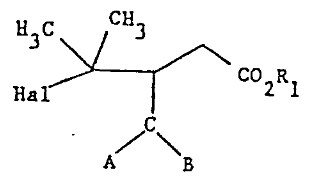

(I)

dans laquelle Hal représente un atome d'halogène, $R_1$ représente un radical alkyle renfermant de 1 à 12 atomes de carbone, et le groupement :

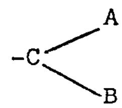

représente soit un groupement

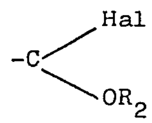

dans lequel Hal est défini comme précédemment et $R_2$ représente un radical alkyle renfermant de 1 à 12 atomes de carbone, soit un groupement

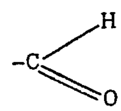

2. Les composés selon la revendication 1, répondant à la formule ($I_A$) :

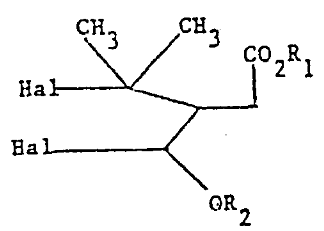

($I_A$)

dans laquelle Hal, $R_1$ et $R_2$ sont définis comme à la revendication 1.

3. Les composés selon la revendication 1, répondant à la formule ($I_B$) :

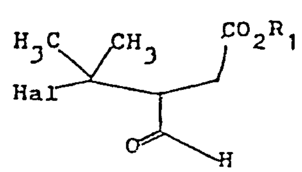

($I_B$)

dans laquelle Hal et $R_1$ sont définis comme à la revendication 1.

4. Les composés de formule (I), tels que définis à la revendication 1, 2 ou 3, pour lesquels Hal représente un atome de chlore.

5. Les composés de formule (I) tels que définis à la revendication 1, 2, 3 ou 4, pour lesquels $R_1$ représente un radical méthyle.

6. Procédé de préparation des composés de formule (I), tels que définis à la revendication 1, caractérisé en ce que l'on soumet un composé de formule (II) :

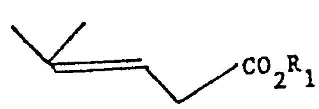

(II)

dans laquelle $R_1$ est défini comme à la revendication 1, à l'action d'un composé de formule (III) :

$(Hal)_2CHOR_2$ (III)

dans laquelle Hal et $R_2$ sont définis comme à la revendication 1, en présence d'un agent acide, pour obtenir un composé de formule ($I_A$) :

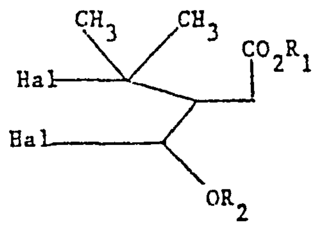

($I_A$)

que l'on soumet le cas échéant à l'action d'un agent d'hydrolyse, pour obtenir un composé de formule ($I_B$) correspondant

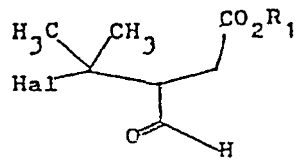

($I_B$)

7. Procédé tel que défini à la revendication 6, caractérisé en ce que l'agent acide utilisé est un acide de Lewis.

8. Procédé tel que défini à la revendication 6 ou 7, caractérisé en ce que l'on fait réagir sur un composé de formule (II)

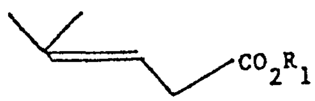

(II)

un composé de formule (III) :

$(Hal)_2CHOR_2$ (III)

dans laquelle $R_2$ a la même signification que $R_1$.

9. Application des composés de formule ($I_A$), telle que définie à la revendication 2, caractérisée en ce que l'on soumet un composé de formule ($I_A$) à l'action d'un agent d'hydrolyse acide, pour obtenir un composé de formule (IV) :

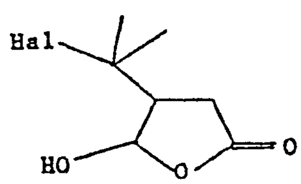

(IV)

dans laquelle Hal est défini comme à la revendication 1.

10. Application des composés de formule ($I_A$), telle que définie à la revendication 2, caractérisée en ce que l'on soumet un composé de formule ($I_A$) à l'action d'un alcool de formule (V) :

$R_2—OH$ (V)

dans laquelle $R_2$ est défini comme à la revendication 1, pour obtenir un composé de formule (VI) :

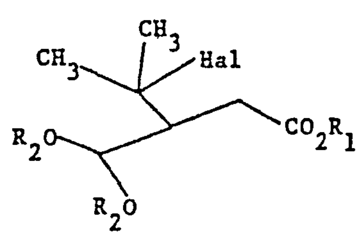

(VI)

que l'on soumet successivement à l'action d'une base, d'un agent de saponification et enfin d'un réactif d'hydrolyse pour obtenir le composé de formule (VII) :

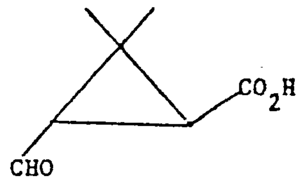

(VII)

de configuration trans.

11. Application des composés de formule ($I_B$), telle que définie à la revendication 3, caractérisée en ce que l'on soumet un composé de formule ($I_B$) à l'action d'un alcool $R_2$—OH dans lequel $R_2$ est défini comme à la revendication 1, pour obtenir un composé de formule (VI) défini à la revendication 10, puis poursuit la synthèse comme décrit à la revendication 10, pour obtenir le composé de formule (VII).

12. A titre de produits chimiques nouveaux, les produits de formule (VI) :

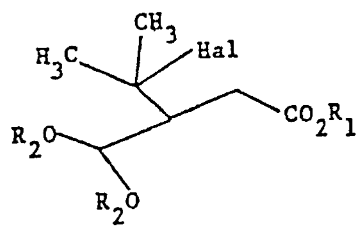

(VI)

dans laquelle Hal, $R_1$ et $R_2$ sont définis comme dans la revendication 1.

**Revendications** (pour l'Etat contractant AT)

1. Procédé de préparation des composés de formule (VII) :

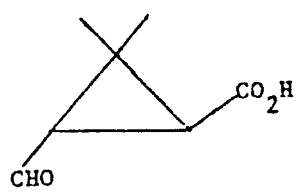

(VII)

de configuration trans, caractérisé en ce que l'on soumet un composé de formule (II) :

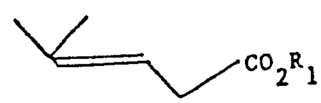

(II)

dans laquelle $R_1$ représente un radical alkyle renfermant de 1 à 12 atomes de carbone, à l'action d'un composé de formule (III) :

$$(Hal)_2CHOR_2 \qquad (III)$$

dans laquelle Hal représente un atome d'halogène et $R_2$ représente un radical alkyle renfermant de 1 à 12 atomes de carbone, en présence d'un agent acide, pour obtenir un composé de formule ($I_A$) :

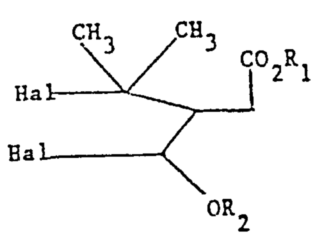

($I_A$)

que l'on soumet à l'action d'un agent d'hydrolyse, pour obtenir un composé de formule ($I_B$) :

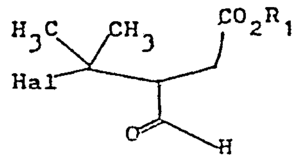

($I_B$)

que l'on soumet à l'action d'un alcool de formule (V) :

$R_2$—OH (V)

dans laquelle $R_2$ est défini comme précédemment, pour obtenir un composé de formule (VI) :

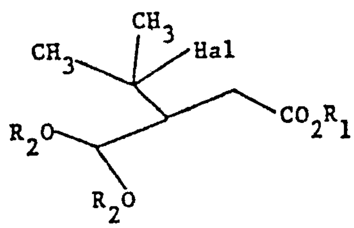

(VI)

que l'on soumet successivement à l'action d'une base, d'un agent de saponification et enfin d'un réactif d'hydrolyse, pour obtenir le composé de formule (VII) :

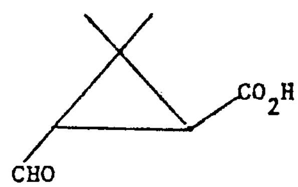

(VII)

de configuration trans.

2. Procédé selon la revendication 1, caractérisé en ce que l'on utilise au départ un composé de formule (II) et un composé de formule (III) dans lesquels Hal représente un atome de chlore et $R_1$ représente un radical méthyle.

3. Procédé selon la revendication 1 ou 2, caractérisé en ce que l'agent acide utilisé est un acide de Lewis.

4. Procédé selon l'une quelconque des revendications 1 à 3, caractérisé en ce que l'on fait réagir sur le composé de formule (II) un composé de formule (III) dans laquelle $R_2$ a la même signification que $R_1$.

5. Procédé selon l'une quelconque des revendications 1 à 4, caractérisé en ce que l'on soumet directement un composé de formule ($I_A$) à l'action d'un alcool de formule (V), pour obtenir un composé de formule (VI).

6. Procédé de préparation des composés de formule (IV) :

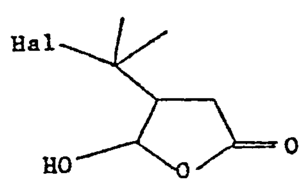

(IV)

dans laquelle Hal est défini comme à la revendication 1, caractérisé en ce que l'on soumet un composé de formule ($I_A$) :

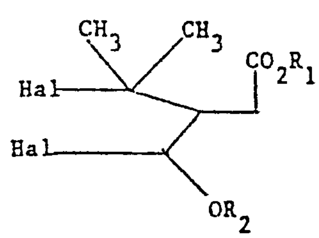

($I_A$)

dans laquelle Hal, $R_1$ et $R_2$ sont définis comme à la revendication 1, à l'action d'un agent d'hydrolyse acide.

**Claims** (for the Contracting States : BE, CH, DE, FR, GB, IT, LI, LU, NL, SE)

1. The compounds with the formula (I) :

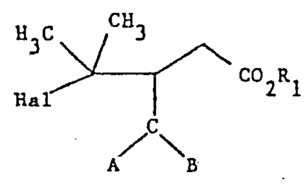

(I)

in which Hal represents a halogen atom, $R_1$ represents an alkyl radical containing 1-12 carbon atoms, and the group :

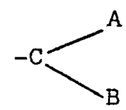

represents either a group :

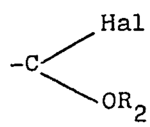

in which Hal is defined as previously and $R_2$ represents an alkyl radical containing 1-12 carbon atoms, or a group :

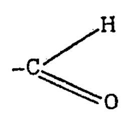

2. The compounds according to Claim 1, corresponding to the formula ($I_A$) :

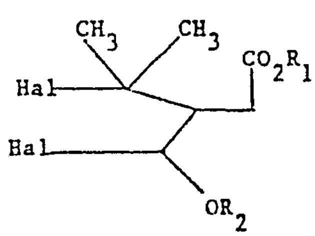

($I_A$)

in which Hal, $R_1$ and $R_2$ are defined as in Claim 1.

3. The compounds according to Claim 1, corresponding to the formula ($I_B$) :

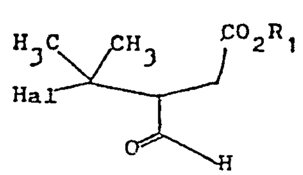

($I_B$)

in which Hal and $R_1$ are defined as in Claim 1.

4. The compounds with the formula (I), as defined in Claim 1, 2 or 3, for which Hal represents a chlorine atom.

5. The compounds with the formula (I) as defined in Claim 1, 2, 3 or 4 for which $R_1$ represents a methyl radical.

6. Preparation process for the compounds with the formula (I), as defined in Claim 1, characterized in that a compound with the formula (II) :

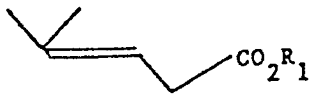

(II)

in which $R_1$ is defined as in Claim 1, is submitted to the action of a compound with the formula (III) :

$$(Hal)_2CHOR_2 \qquad (III)$$

in which Hal and $R_2$ are defined as in Claim 1, in the presence of an acid agent, so as to obtain a compound with the formula ($I_A$) :

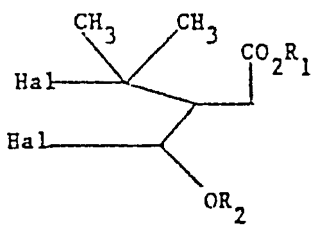

($I_A$)

which, if applicable, is submitted to the action of a hydrolyzing agent, so as to obtain a corresponding compound with the formula ($I_B$) :

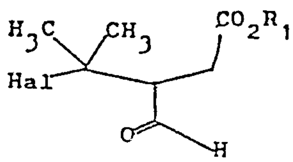

($I_B$)

7. Process as defined in Claim 6, characterized in that the acid agent utilized is a Lewis acid.

8. Process as defined in Claim 6 or 7, characterized in that a compound with the formula (III) :

$$(Hal)_2CHOR_2 \qquad (III)$$

in which $R_2$ has the same significance as $R_1$, is made to react on a compound with the formula (II) :

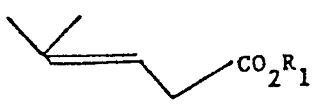

(II)

9. Use of the compounds with the formula ($I_A$), as defined in Claim 2, characterized in that a compound with the formula ($I_A$) is submitted to the action of an acid hydrolyzing agent, so as to obtain a compound with the formula (IV) :

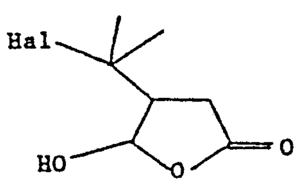

(IV)

in which Hal is defined as in Claim 1.

10. Use of the compounds with the formula ($I_A$), as defined in Claim 2, characterized in that a compound with the formula ($I_A$) is submitted to the action of an alcohol with the formula (V) :

$$R_2—OH \qquad (V)$$

in which $R_2$ is defined as in Claim 1, so as to obtain a compound with the formula (VI) :

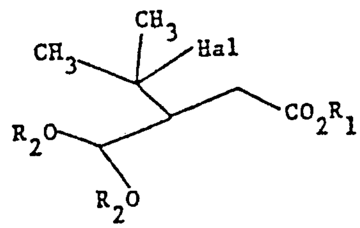

(VI)

which is submitted, successively, to the action of a base, of a saponification agent and, finally, of a hydrolyzing reagent so as to obtain the compound with the formula (VII) :

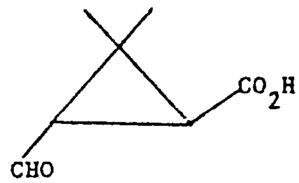

(VII)

of trans configuration.

11. Use of the compounds with the formula ($I_B$), as described in Claim 3, characterized in that a compound with the formula ($I_B$) is submitted to the action of an $R_2$—OH alcohol in which $R_2$ is defined as in Claim 1, so as to obtain a compound with the formula (VI) defined in Claim 10, then the synthesis as described in Claim 10 is continued, so as to obtain the compound with the formula (VII).

12. As new chemical products, the products with the formula (VI) :

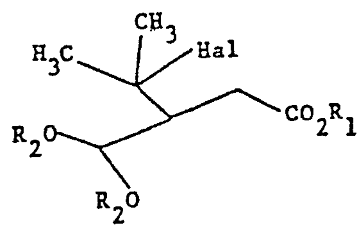

(VI)

in which Hal, $R_1$ and $R_2$ are defined as in Claim 1.

**Claims** (for the Contracting State AT)

1. Preparation process for the compounds with the formula (VII) :

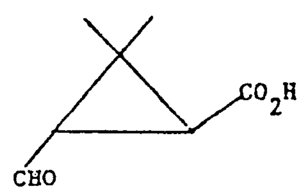

(VII)

of trans configuration, characterized in that a compound with the formula (II) :

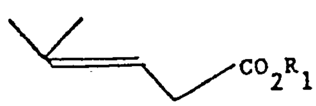

(II)

in which $R_1$ represents an alkyl radical containing 1-12 carbon atoms, is submitted to the action of a compound with the formula (III) :

$$(Hal)_2CHOR_2 \quad (III)$$

in which Hal represents a halogen atom and $R_2$ represents an alkyl radical containing 1-12 carbon atoms, in the presence of an acid agent, so as to obtain a compound with the formula ($I_A$) :

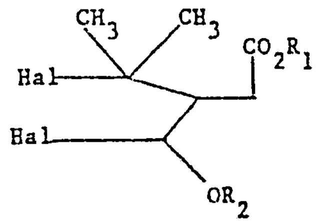

($I_A$)

which is submitted to the action of a hydrolyzing agent, so as to obtain a compound with the formula ($I_B$) :

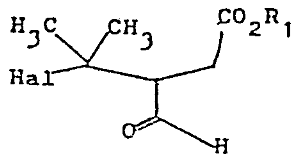

($I_B$)

which is submitted to the action of an alcohol with the formula (V) :

$$R_2—OH \qquad (V)$$

in which $R_2$ is defined as previously, so as to obtain a compound with the formula (VI) :

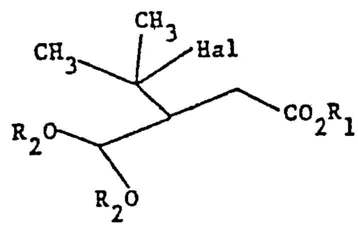

(VI)

which is submitted, successively, to the action of a base, of a saponification agent and, finally, of a hydrolyzing reagent, so as to obtain the compound with the formula (VII) :

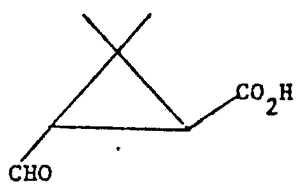

(VII)

of trans configuration.

2. Process according to Claim 1, characterized in that a compound with the formula (II) and a compound the formula (III) in which Hal represents a chlorine atom and $R_1$ represents a methyl radical are utilized at the start.

3. Process according to Claim 1 or 2, characterized in that the acid agent utilized is a Lewis acid.

4. Process according to any one of the Claims 1-3, characterized in that a compound with the formula (III) in which $R_2$ has the same significance as $R_1$ is made to react on a compound with the formula (II).

5. Process according to any one of the Claims 1-4, characterized in that a compound with the formula ($I_A$) is directly submitted to the action of an alcohol with the formula (V), so as to obtain a compound with the formula (VI).

6. Preparation process for the compounds with the formula (IV) :

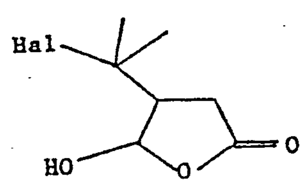

(IV)

in which Hal is defined as in Claim 1, characterized in that a compound with the formula ($I_A$) :

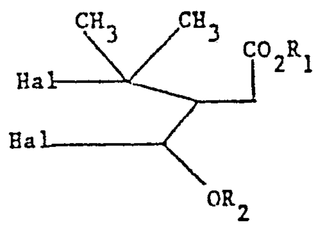

$(I_A)$

in which Hal, $R_1$ and $R_2$ are defined as in Claim 1, is submitted to the action of an acid hydrolyzing agent.

**Ansprüche** (für die Vertragsstaaten : BE, CH, DE, FR, GB, IT, LI, LU, NL, SE)

1. Verbindungen der Formel (I)

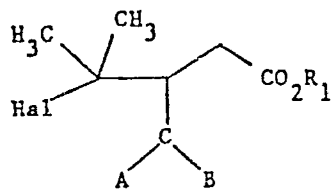

(I)

worin Hal ein Halogenatom bedeutet, $R_1$ einen Alkylrest mit 1 bis 12 Kohlenstoffatomen bedeutet und die Gruppe

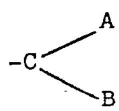

entweder eine Gruppe

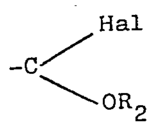

worin Hal wie vorstehend definiert ist und $R_2$ einen Alkylrest mit 1 bis 12 Kohlenstoffatomen bedeutet, oder eine Gruppe

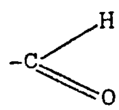

darstellt.

2. Verbindungen gemäß Anspruch 1 der Formel $(I_A)$

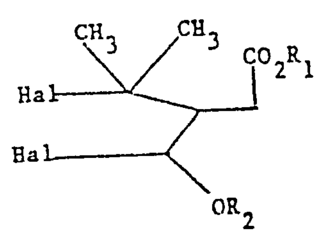

$(I_A)$

worin Hal, $R_1$ und $R_2$ wie in Anspruch 1 definiert sind.

3. Verbindungen gemäß Anspruch 1 der Formel $(I_B)$

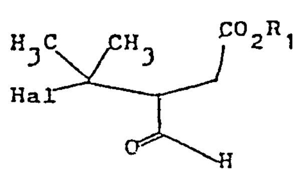

$(I_B)$

worin Hal und $R_1$ wie in Anspruch 1 definiert sind.

4. Verbindungen der Formel (I) gemäß Anspruch 1, 2 oder 3, worin Hal ein Chloratom bedeutet.

5. Verbindungen der Formel (I) gemäß Anspruch 1, 2, 3 oder 4, worin $R_1$ einen Methylrest bedeutet.

6. Verfahren zur Herstellung der Verbindungen der Formel (I) gemäß Anspruch 1, dadurch gekennzeichnet, daß man eine Verbindung der Formel (II)

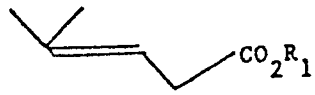

(II)

worin $R_1$ wie in Anspruch 1 definiert ist, der Einwirkung einer Verbindung der Formel (III)

$$(Hal)_2CHOR_2 \quad (III)$$

worin Hal und $R_2$ wie in Anspruch 1 definiert sind, in Gegenwart eines sauren Mittels unterzieht, um eine Verbindung der Formel ($I_A$)

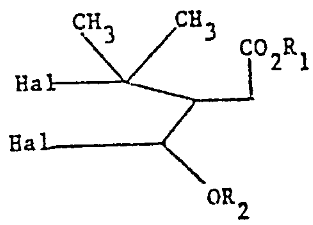

($I_A$)

zu erhalten, die man gegebenenfalls der Einwirkung eines Hydrolysemittels unterzieht, um eine entsprechende Verbindung der Formel ($I_B$)

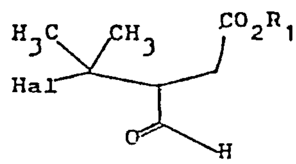

($I_B$)

zu erhalten.

7. Verfahren gemäß Anspruch 6, dadurch gekennzeichnet, daß das verwendete, saure Mittel eine Lewissäure ist.

8. Verfahren gemäß Anspruch 6 oder 7, dadurch gekennzeichnet, daß man mit einer Verbindung der Formel (II)

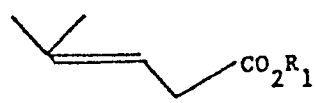

(II)

eine Verbindung der Formel (III)

$$(Hal)_2CHOR_2 \quad (III)$$

worin $R_2$ die gleiche Bedeutung wie $R_1$ besitzt, reagieren läßt.

9. Verwendung der Verbindungen der Formel ($I_A$) gemäß Anspruch 2, dadurch gekennzeichnet, daß man eine Verbindung der Formel ($I_A$) der Einwirkung eines sauren Hydrolysemittels unterzieht, um eine Verbindung der Formel (IV)

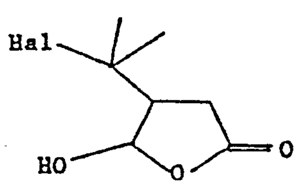

(IV)

worin Hal wie in Anspruch 1 definiert ist, zu erhalten.

10. Verwendung der Verbindungen der Formel ($I_A$) gemäß Anspruch 2, dadurch gekennzeichnet, daß man die Verbindung der Formel ($I_A$) der Einwirkung eines Alkohols der Formel (V)

$$R_2—OH \quad (V)$$

unterzieht, worin $R_2$ wie in Anspruch 1 definiert ist, um eine Verbindung der Formel (VI)

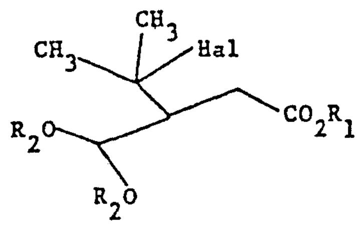

(VI)

zu erhalten, die man nacheinander der Einwirkung einer Base, eines Verseifungsmittels und schließlich eines Hydrolysereagens unterzieht, um die Verbindung der Formel (VII)

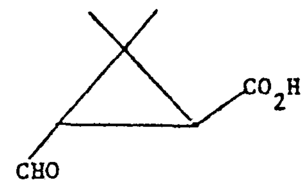

(VII)

mit trans-Konfiguration zu erhalten.

11. Verwendung der Verbindungen der Formel ($I_B$) gemäß Anspruch 3, dadurch gekennzeichnet, daß man eine Verbindung der Formel ($I_B$) der Einwirkung eines Alkohols $R_2$—OH unterzieht, worin $R_2$ wie in Anspruch 1 definiert ist, um eine Verbindung der Formel (VI), die wie in Anspruch 10 definiert ist, zu erhalten, und danach die Synthese wie in Anspruch 10 beschrieben fortsetzt, um die Verbindung der Formel (VII) zu erhalten.

12. Als neue chemische Produkte die Produkte der Formel (VI)

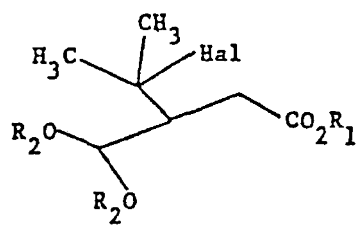

(VI)

worin Hal, $R_1$ und $R_2$ wie in Anspruch 1 definiert sind.

**Ansprüche** (für den Vertragsstaat AT)

1. Verfahren zur Herstellung von Verbindungen der Formel (VII)

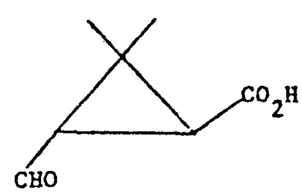

(VII)

mit trans-Konfiguration, dadurch gekennzeichnet, daß man eine Verbindung der Formel (II)

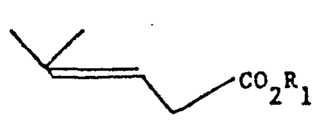

(II)

worin $R_1$ einen Alkylrest mit 1 bis 12 Kohlenstoffatomen bedeutet, der Einwirkung einer Verbindung der Formel (III)

$$(Hal)_2CHOR_2 \quad (III)$$

worin Hal ein Halogenatom bedeutet und $R_2$ einen Alkylrest mit 1 bis 12 Kohlenstoffatomen darstellt, in Gegenwart eines sauren Mittels unterzieht, um eine Verbindung der Formel ($I_A$)

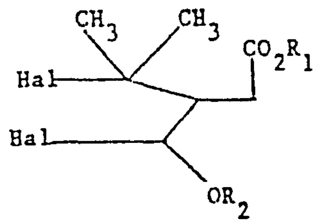

($I_A$)

zu erhalten, die man der Einwirkung eines Hydrolysemittels unterzieht, um eine Verbindung der Formel ($I_B$)

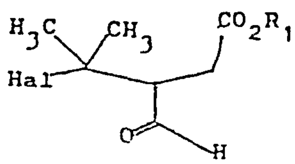

($I_B$)

zu erhalten, die man der Einwirkung eines Alkohols der Formel (V)

$$R_2\text{—OH} \qquad (V)$$

unterzieht, worin $R_2$ wie vorstehend definiert ist, um eine Verbindung der formel (VI)

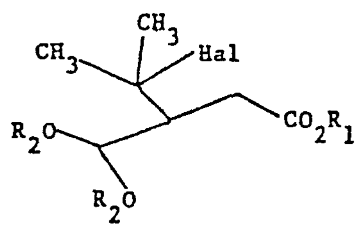

(VI)

zu erhalten, die man nacheinander der Einwirkung einer Base, eines Verseifungsmittels und schließlich eines Hydrolysereagens unterzieht, um die Verbindung der Formel (VII)

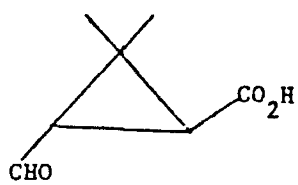

(VII)

mit trans-Konfiguration zu erhalten.

2. Verfahren gemäß Anspruch 1, dadurch gekennzeichnet, daß man von einer Verbindung der Formel (II) und einer Verbindung der Formel (III) ausgeht, worin Hal ein Chloratom bedeutet und $R_1$ einen Methylrest darstellt.

3. Verfahren gemäß Anspruch 1 oder 2, dadurch gekennzeichnet, daß das verwendete saure Mittel eine Lewissäure ist.

4. Verfahren gemäß einem der Ansprüche 1 bis 3, dadurch gekennzeichnet, daß man mit der Verbindung der Formel (II) eine Verbindung der Formel (III) reagieren läßt, worin $R_2$ die gleiche Bedeutung wie $R_1$ besitzt.

5. Verfahren gemäß einem der Ansprüche 1 bis 4, dadurch gekennzeichnet, daß man direkt eine Verbindung der Formel ($I_A$) der Einwirkung eines Alkohols der Formel (V) unterzieht, um eine Verbindung der Formel (VI) zu erhalten.

6. Verfahren zur Herstellung der Verbindungen der Formel (IV)

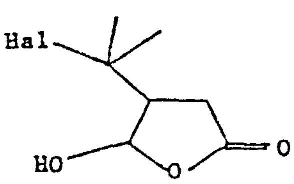

(IV)

worin Hal wie in Anspruch 1 definiert ist, dadurch gekennzeichnet, daß man eine Verbindung der Formel ($I_A$)

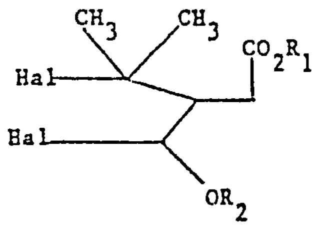

($I_A$)

worin Hal, $R_1$ und $R_2$ wie in Anspruch 1 definiert sind, der Einwirkung eines sauren Hydrolysemittels unterzieht.